# EUROPEAN PATENT SPECIFICATION

(11) **EP 4 079 244 B1**
(45) Date of publication and mention of the grant of the patent: **22.10.2025**
(21) Application number: 22168805.4
(22) Date of filing: 19.04.2022
(51) Int. Cl.: A61B 18/12, A61B 18/14, A61B 18/00

(54) **IRE ABLATION SYSTEMS AND PROTOCOLS USING A BASKET CATHETER**
IRE-ABLATIONSSYSTEME UND -PROTOKOLLE UNTER VERWENDUNG EINES KORBKATHETERS
SYSTÈMES ET PROTOCOLES D'ABLATION D'ÉLECTROPORATION IRRÉVERSIBLE UTILISANT UN CATHÉTER À PANIER

(30) Priority: 19.04.2021 US 202117234625
(43) Date of publication of application: 26.10.2022
(73) Proprietor: Biosense Webster (Israel) Ltd., Yokneam 2066717 (IL)
(72) Inventor: GOVARI, Assaf, 2066717 Yokneam (IL); ALTMANN, Andres Claudio, 2066717 Yokneam (IL); SHAMIS, Yuri, 2066717 Yokneam (IL); MARZIANO, Lilah, 2066717 Yokneam (IL)
(74) Representative: Carpmaels & Ransford LLP

(56) References cited:
- WO-A1-2020/251857
- CN-A- 111 728 693
- US-A1- 2020 289 197

## Description

### FIELD OF THE INVENTION

The present invention relates generally to invasive medical equipment, and particularly to apparatus for ablating tissue within the body.

### BACKGROUND

Irreversible electroporation (IRE) is a soft tissue ablation technique that applies short pulses of strong electrical fields to create permanent and hence lethal nanopores in the cell membrane, thus disrupting the cellular homeostasis (internal physical and chemical conditions). Cell death following IRE results from apoptosis (programmed cell death) and not necrosis (cell injury, which results in the destruction of a cell through the action of its own enzymes) as in other thermal and radiation-based ablation techniques. IRE is commonly used in tumor ablation in regions where precision and conservation of the extracellular matrix, blood flow and nerves are of importance.

Some electroporation techniques use basket catheters, in which electrodes are mounted on flexible spines at the distal end of the catheter, and the spines bend outward to form a basket-like shape and contact tissue within a body cavity. For example, U.S. Patent Application Publication 2020/0289197 describes devices and methods for electroporation ablation therapy, with the device including a set of splines coupled to a catheter for medical ablation therapy. Each spline of the set of splines may include a set of electrodes formed on that spline. The set of splines may be configured for translation to transition between a first configuration and a second configuration.

US 2020/289197 A1 discloses a device for electroporation ablation therapy including a set of splines coupled to a catheter for medical ablation therapy. Each spline of the set of splines may include a set of electrodes formed on that spline.

CN 111728693 discloses a system for treating arrhythmia by adopting a pulsed electric field ablation technology. The system comprises a voltage pulse system console, a pace-making and ECG unit and anablation catheter.

### SUMMARY

The present invention is defined in the appended claims. Embodiments are provided in the dependent claims. In the following all methods of treatment are not claimed and are disclosed for illustrative purposes only.

The present invention will be more fully understood from the following detailed description of the embodiments thereof, taken together with the drawings in which:

### BRIEF DESCRIPTION OF THE DRAWINGS

Fig. 1 is a schematic pictorial illustration of a system used in an IRE ablation procedure;
Fig. 2 is a schematic pictorial illustration of the distal end of a basket catheter used in IRE;
Fig. 3 is a flow chart, which schematically illustrates a method for IRE;
Fig. 4 is a schematic pictorial illustration of the distal end of a basket catheter used in IRE.

### DETAILED DESCRIPTION OF EMBODIMENTS

### OVERVIEW

IRE is a predominantly non-thermal process, which causes an increase of the tissue temperature by, at most, a few degrees for a few seconds. It thus differs from RF (radio frequency) ablation, which raises the tissue temperature by between 20 and 70°C and destroys cells through heating. IRE typically utilizes biphasic pulses, i.e., combinations of positive and negative electrical pulses, in order to avoid muscle contraction due to a cumulative DC voltage. The pulses are applied, for example, between two bipolar electrodes of a catheter.

Because IRE is an electrical, rather than a thermal, process, different cells will respond differently to the electric field generated by the IRE depending, for example, on the different shapes, sizes, and orientations of the cells. The effectiveness of IRE ablation may therefore depend not only on the amplitudes of the electrical pulses that are applied to the tissue, but also on the geometrical directions. To ensure effective ablation, it is therefore desirable that electric fields be applied to the target tissue along multiple different directions. This sort of multi-directional approach can be difficult or impossible to implement using a conventional focal catheter, however, or even using basket catheters that are known in the art.

Embodiments of the present invention that are described herein provide basket catheters with a three-dimensional arrangement of electrodes that can be used to address this problem. Specifically, the disclosed embodiments provide a probe, such as a catheter, comprising an insertion tube for insertion into a body cavity, such as a chamber of the heart, and a basket assembly, whose proximal end is connected distally to the insertion tube. The basket assembly comprises resilient spines, which bow radially outward around the axis of the basket assembly and are conjoined at the distal end of the basket assembly. One or more radial electrodes are disposed on each of the spines, and an axial electrode is disposed at the distal end of the basket assembly, i.e., on the axis of the basket assembly at its distal tip.

An electrical signal generator applies IRE pulses to the electrodes, including the axial electrode. (The term "IRE pulses" is used in the present description and in the claims to mean electrical pulses having an amplitude sufficient to cause IRE in the tissue contacted by the electrodes.) The presence of the axial electrode makes it possible for the pulses to be applied in a bipolar mode in multiple different directions, both between different pairs of the radial electrodes and between the radial electrodes and the axial electrode. Additionally or alternatively, the electrical signal generator can apply the pulses in a unipolar mode between any of the radial electrodes and/or the axial electrode and a common electrode, such as a back patch, that is separate from the probe. Thus, using this sort of basket catheter with an axial (tip) electrode, IRE pulses can be applied to the target tissue in multiple different directions, while the basket is held stationary against the tissue.

To ablate a particular region of tissue effectively using this sort of catheter, the IRE pulses should be applied sequentially to different electrodes and pairs of electrodes on the catheter, such that the electric field reaches all areas of the tissue in multiple different field orientations. It is also desirable that the sequence of actuation of the electrodes by the IRE pulses be chosen so that the electrical power and residual heat are dissipated evenly between the electrodes. Although this sort of selection can be performed manually by the practitioner carrying out the ablation procedure, it is time-consuming, tedious, and error prone.

Therefore, predefined IRE ablation protocols that the practitioner can choose are provided according to the present invention.

Each protocol designates a sequence of electrodes or pairs of electrodes to which IRE pulses are to be applied in order to cover the region to be ablated with all the desired electric field orientations. The protocols may also specify the IRE pulse parameters for each electrode or pair of electrodes in the sequence. Both unipolar and bipolar ablation protocols may be defined in this manner. For effective dissipation of electrical and thermal energy, the protocols specify the sequence of electrodes such that the IRE pulses are not applied in immediate succession to radial electrodes on mutually adjacent spines.

In bipolar protocols, for example, IRE pulses are applied in a bipolar mode between pairs of the radial electrodes and between each of the radial electrodes and the axial electrode. In some bipolar protocols, the pulses are applied simultaneously between two or more of the radial electrodes and the axial electrode. The IRE pulses are applied between pairs of the radial electrodes on different spines that are not mutually adjacent. When the basket assembly comprises two or more radial electrodes on each of the spines, the protocol may specify that the IRE pulses be applied to only a subset of the radial electrodes on each spine, such as the most distal radial electrode or the most proximal.

### SYSTEM DESCRIPTION

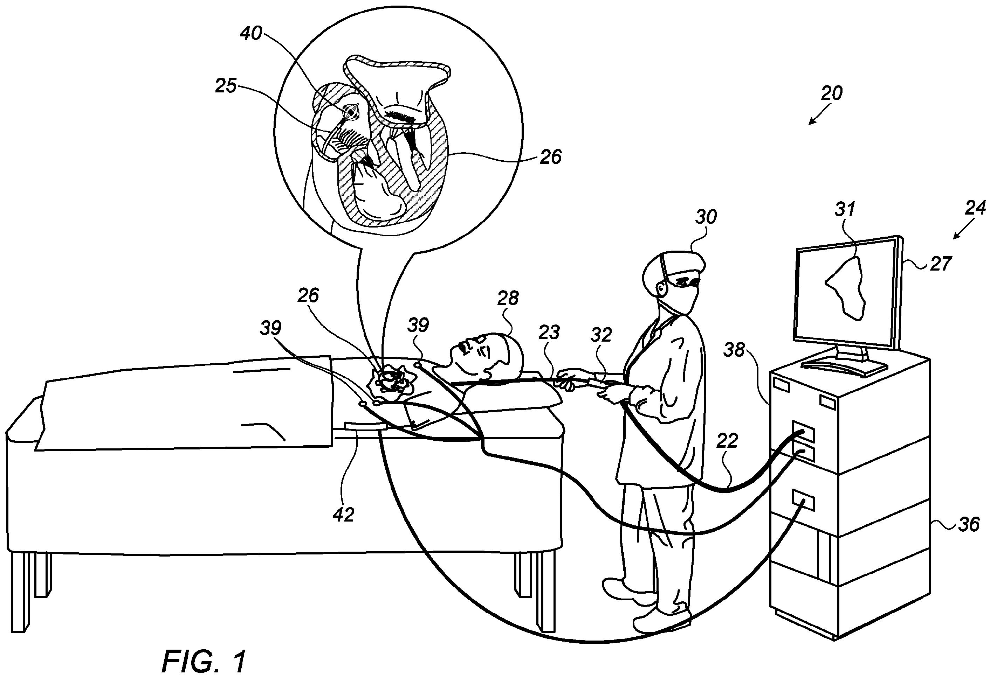

Fig. 1 is a schematic pictorial illustration of a system 20 used in an IRE ablation procedure. Elements of system 20 may be based on components of the CARTO^{®} system, produced by Biosense Webster, Inc. (Irvine, California).

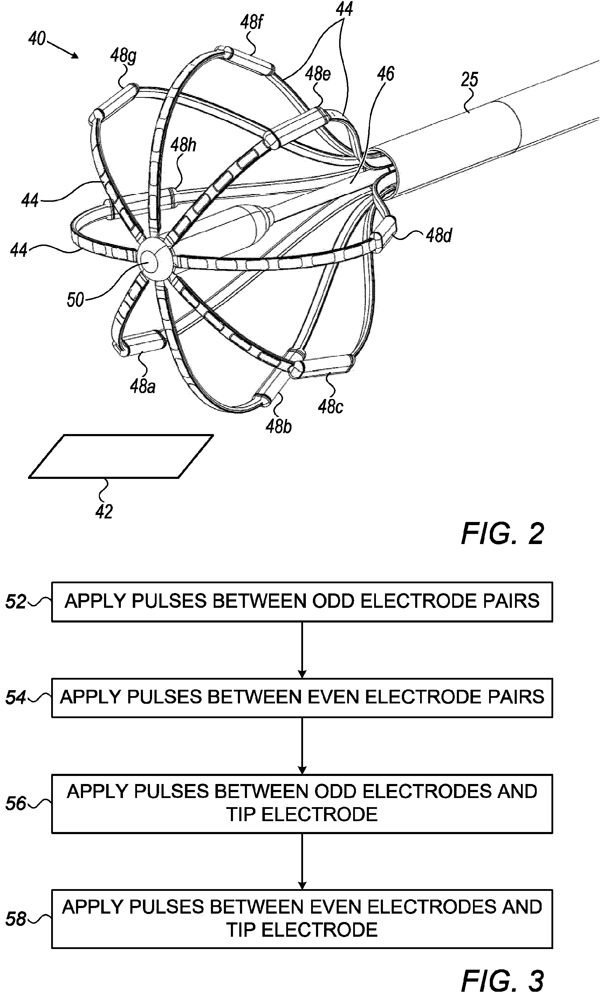

A physician 30 navigates a catheter 22 through the vascular system of a patient 28 into a chamber of a heart 26 of the patient, and then deploys a basket assembly 40 (shown in detail in Fig. 2) at the distal end of the catheter. The proximal end of basket assembly 40 is connected to the distal end of an insertion tube 25, which physician 30 steers using a manipulator 32 near the proximal end of catheter 22. Basket assembly 40 is inserted in a collapsed configuration through a sheath 23, which passes through the vascular system of patient 28 into the heart chamber where the IRE procedure is to be performed, and is then deployed from the sheath and allowed to expand within the chamber. Catheter 22 is connected at its proximal end to a control console 24. A display 27 on console 24 may present a map 31 or other image of the heart chamber with an icon showing the location of basket assembly 40 in order to assist physician 30 in positioning the basket assembly at the target location for the IRE ablation procedure.

Once basket assembly 40 is properly deployed and positioned in heart 26, physician 30 actuates an electrical signal generator 38 in console 24 to apply sequences of IRE pulses to the electrodes on the basket assembly, under the control of a processor 36. The IRE pulses are typically applied in a bipolar mode, between pairs of the electrodes on basket assembly 40. Additionally or alternatively, the pulses may be applied between the electrodes on basket assembly 40 and a separate common electrode, for example a conductive back patch 42, which is applied to the patient's skin.

To perform IRE, electrical signal generator 38 typically applies sequences of pulses to the electrodes with an amplitude of at least approximately 200 V, while the duration of each pulse is less than approximately 20 µs. The sequences of the pulses comprise biphasic pairs of pulses, meaning that each pair comprises a positive pulse and a negative pulse. (The terms "positive" and "negative" are used arbitrarily to indicate the opposing electrical polarities of the pulses.) An electrical signal generator capable of outputting this sort of IRE pulse sequences, while switching rapidly between different pairs of electrodes, is described, for example, in U.S. Patent Application 16/701,989, filed December 3, 2019.

Processor 36 directs electrical signal generator 38 to switch among the electrodes and apply the pulses in accordance with a predefined protocol, such as one or more of the protocols described further hereinbelow. Physician 30 may select the protocol to apply using controls on console 24. For example, the physician or an assistant may use a touch screen functionality of display 27 on console 24 to interact with processor 36. Alternatively or additionally, the physician or an assistant may operate the controls in order to select the pulse parameters and electrodes manually.

Typically, catheter 22 comprises one or more position sensors (not shown in the figures), which output position signals that are indicative of the position (location and orientation) of basket assembly 40. For example, basket assembly 40 may incorporates one or more magnetic sensors, which output electrical signals in response to an applied magnetic field. Processor 36 receives and processes the signals in order to find the location and orientation coordinates of basket assembly 40, using techniques that are known in the art and are implemented, for example, in the above-mentioned Carto system. Alternatively or additionally, system 20 may apply other position-sensing technologies in order to find the coordinates of basket assembly 40. For example, processor 36 may sense the impedances between the electrodes on basket assembly 40 and body-surface electrodes 39, which are applied to the chest of patient 28, and may convert the impedances into location coordinates using techniques that are likewise known in the art. In any case, processor 41 uses the coordinates in displaying the location of basket assembly 40 on map 31.

Alternatively, catheter 22 and the IRE ablation protocols that are described herein may be used without the benefit of position sensing. In such embodiments, for example, fluoroscopy and/or other imaging techniques may be used to ascertain the location of basket assembly 40 in heart 26.

The system configuration that is shown in Fig. 1 is presented by way of example for conceptual clarity in understanding the operation of embodiments of the present invention. For the sake of simplicity, Fig. 1 shows only the elements of system 20 that are specifically related to the disclosed techniques. The remaining elements of the system will be apparent to those skilled in the art, who will likewise understand that the principles of the present invention may be implemented in other medical therapeutic systems, using other components, as far as they fall within the scope of the claims.

### BASKET ASSEMBLIES AND IRE PROTOCOLS

Fig. 2 is a schematic pictorial illustration of basket assembly 40, in accordance with an embodiment of the invention. Basket assembly 40 comprises multiple resilient spines 44, with one radial electrode 48a, 48b, 48c, 48d, 48e, 48f, 48g, 48h disposed on each of the spines. (These electrodes are referred to collectively as radial electrodes 48.) An axial electrode 50 is disposed at the tip of the basket, i.e., at the distal end of the basket assembly. Although basket assembly 40 is shown as comprising eight spines 44, in alternative embodiments the basket assembly may comprise a larger or smaller number of spines.

Spines 44 typically comprise a suitable, resilient metal or plastic material, for example. The proximal tips of spines 44 are coupled together at a at the proximal end of the basket assembly, where the basket assembly connects to the distal end of insertion tube 25. The distal tips of spines 44 are likewise coupled together (using suitable joining or coupling technologies) at the distal end of the basket assembly. The spines bow radially outward when basket assembly 40 is deployed from sheath 23 into the heart chamber. Physician 30 then manipulates catheter 22 so that electrodes 48 and 50 contact the myocardial tissue at the target location in the heart chamber.

Basket assembly 40 may comprise other components, as well (not shown in the figures), such as ultrasound transducers, contact force sensors, and temperature sensors. Electrodes 48 and 50, as well as these other components, are connected to wires (not shown) running through insertion tube 25 to the proximal end of catheter 22, where they connect to appropriate circuitry in console 24. Further details of the construction of spines 44, including mechanical and electrical attachment of electrodes 48 and 50 to basket assembly 40, are presented in the above-mentioned U.S. Provisional Patent Application 63/076,614. The surface mounted electrodes 48a, 48b, 48c ... 48h extend beyond the surface of the spines 44 (i.e., bulging beyond the surface boundary of the spine) to ensure that the respective electrodes 48a-48h are in sufficient tissue contact. Alternatively, other designs of the electrodes and spines may be used, as will be apparent to those skilled in the art after reading the present description.

Various mechanisms can be used to collapse basket assembly 40 as it passes through sheath 23 and to cause spines 44 to bow radially outward when deployed from the sheath, so as to assume the expanded state that is shown in Fig. 2. In the present example, basket assembly 40 has a stable collapsed state, in which spines 44 straighten along the axial direction (i.e., parallel to the longitudinal axis of insertion tube 25). A puller 46, such as a metal or plastic rod or wire, is attached to the distal end of basket assembly 40, where axial electrode 50 is located and spines 44 are conjoined. Puller 46 is slidably disposed within insertion tube 25. To deploy the basket assembly, puller 46 is pulled in the proximal direction through the insertion tube, thus causing spines 44 to bow radially outward. It is noted that while electrode 50 is illustrated in one embodiment as being a dome shaped electrode, it is within the scope of the claimed invention to utilize a cylindrical electrode 51 instead of the dome shaped electrode 50 as well as both dome electrode 50 and cylindrical electrode 51.

Fig. 3 is a flow chart that schematically illustrates a method for IRE. The method illustrated in the figure implements a protocol for bipolar ablation, in which IRE pulses are applied between different pairs of electrodes 48 and 50. For the sake of convenience, radial electrodes 48a, 48c, 48e and 48g are referred to as the "odd" electrodes in this context, while radial electrodes 48b, 48d, 48f and 48h are referred to as the "even" electrodes.

In an initial radial ablation step 52, electrical signal generator 38 applies IRE pulses in alternation between pairs of the odd radial electrodes, for example between electrodes 48a and 48c, then between electrodes 48c and 48e, then between electrodes 48e and 48g, and finally between electrodes 48g and 48a. In a second radial ablation step 54, electrical signal generator 38 applies IRE pulses in alternation between pairs of the even radial electrodes, for example between electrodes 48b and 48d, then between electrodes 48d and 48f, then between electrodes 48f and 48h, and finally between electrodes 48h and 48b.

After completing the radial ablation steps, electrical signal generator 38 applies IRE pulses between the odd radial electrodes 48 and axial electrode 50, at an initial axial ablation step 56. Thus, pulses are applied between electrodes 48a and 50, then between electrodes 48c and 50, then between electrodes 48e and 50, and finally between electrodes 48g and 50. In a second axial ablation step 58, electrical signal generator 38 applies IRE pulses between electrodes 48b and 50, then between electrodes 48d and 50, then between electrodes 48f and 50, and finally between electrodes 48h and 50. Alternatively, in steps 56 and 58, IRE pulses may be applied simultaneously between two or more of radial electrodes 48 and axial electrode 50.

The above order of actuation of the electrodes is described by way of example. Alternative protocols, with different orders of electrode actuation, will be apparent to those skilled in the art after reading the present description and are considered to be within the scope of the present invention, as far as they fall within the scope of the claims.

In an alternative protocol, electrical signal generator 38 applies IRE pulses to electrodes 48 and 50 in a unipolar mode, for example between electrodes 48 and 50 and back patch 42. The electrodes to be actuated are chosen depending on the area of the myocardial tissue that is to be ablated. The electrodes may be actuated either individually, or simultaneously in groups of two or more, or all at once. Alternatively, the unipolar IRE pulses may be applied between electrodes 48 and 50 on basket assembly 40 and a common electrode inside the body, for example an electrode on another catheter, as long as this common internal electrode is sufficiently large to convey the IRE power.

As noted earlier, the IRE pulses applied to electrodes 48 and 50 typically have an amplitude of at least approximately 200 V, and a duration of each of the pulses is less than approximately 20 µs. In one embodiment, each selected set of electrodes is energized in bursts, wherein each burst comprises a sequence of pulses, for example ten biphasic pulses of amplitude 1000 V and pulse duration 2 µs, with a delay of 5 ms from pulse to pulse. Twenty bursts of this sort may be applied to each set of the electrodes. Alternatively, smaller or larger numbers of bursts may be applied, and different pulse parameters may be defined.

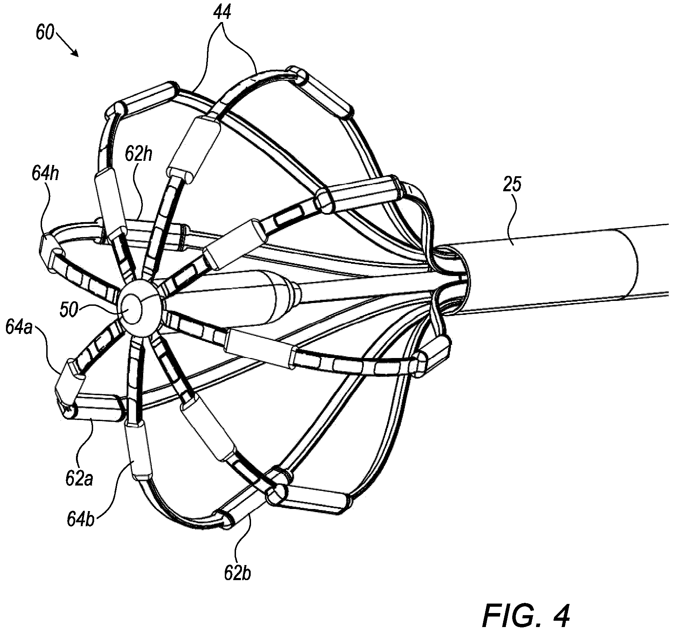

Fig. 4 is a schematic pictorial illustration of a basket assembly 60 at the distal end of a catheter used in IRE. Basket assembly 60 is similar in construction and functionality to basket assembly 40, as described above, except that multiple radial electrodes are disposed on each spine 44, including proximal radial electrodes 62a, 62b, ..., 62h and distal radial electrodes 64a, 64b, ..., 64h. In the pictured embodiment, the locations of the radial electrodes along spines 44 are longitudinally staggered, i.e., different distal radial electrodes, such as electrodes 64a and 64b, are located at different distances from the distal ends of the respective spines 44 on which they are mounted; and proximal radial electrodes 62a and 62b are similarly mounted at different distances from the distal ends of the spines. This sort of staggering can be useful in varying the locations and directions across which IRE pulses are applied to the tissue with which basket assembly 60 is in contact. Alternatively, the basket assembly may comprise a larger number of radial electrodes, which may be staggered or uniformly positioned along the respective spines.

The same sorts of protocols, including both bipolar and unipolar protocols, may be applied in actuating the electrodes on basket assembly 60 as were described above with respect to basket assembly 40. The protocols may be modified to include actuation of all of radial electrodes 62a, 62b, ..., 62h and 64a, 64b, ..., 64h. Alternatively, only a subset of the radial electrodes may be actuated, depending on the therapeutic plan and contact between the electrodes and the target tissue. For example, IRE pulses may be applied in a bipolar mode only between distal radial electrodes 64a, 64b, ..., 64h and axial electrode 50.

It will be appreciated that the embodiments described above are cited by way of example, and that the present invention is not limited to what has been particularly shown and described hereinabove. Rather, the disclosure includes both combinations and subcombinations of the various features described hereinabove, as well as variations and modifications thereof which would occur to persons skilled in the art upon reading the foregoing description. The invention is defined in the appended claims.

### ASPECTS OF THE INVENTION

1. A method for medical treatment, comprising:
   providing a basket assembly for insertion into a body cavity of a patient, the basket assembly comprising a plurality of resilient spines, which are configured to bow radially outward around a longitudinal axis of the basket assembly and are conjoined at a distal end of the basket assembly, and a plurality of electrodes, which are configured to contact tissue in the body cavity and comprise radial electrodes disposed on the spines and an axial electrode disposed on the longitudinal axis of the basket assembly; and
   applying to the electrodes, including the axial electrode, pulses having an amplitude sufficient to cause irreversible electroporation (IRE) in the tissue contacted by the electrodes.
2. The method according to aspect 1, wherein the spines have respective proximal and distal tips, wherein the proximal tips of the spines are coupled together at a at a proximal end of the basket assembly, and the distal tips of the spines are coupled together at a at a distal end of the basket assembly, and the spines bow radially outward when the basket assembly is deployed in the body cavity, whereby the radial electrodes contact the tissue in the body cavity.
3. The method according to aspect 2, wherein the basket assembly comprises a stable collapsed state, and wherein providing the basket assembly comprises a attaching a puller to the distal end of the basket assembly and slidably disposed within the insertion tube, so that the spines bow radially outward in response to pulling the puller in a proximal direction through the insertion tube.
4. The method according to aspect 1, wherein the insertion tube comprises a flexible catheter configured for insertion into a chamber of a heart of the patient, and the electrodes are configured to contact and apply the electrical signals to myocardial tissue within the chamber.
5. The method according to aspect 1, wherein the radial electrodes comprise a single respective radial electrode on each of the spines.
6. The method according to aspect 1, wherein the radial electrodes comprise multiple radial electrodes on each of the spines in respective locations that are longitudinally staggered among the spines.
7. The method according to aspect 1, wherein applying the pulses comprises applying a sequence of the pulses having an amplitude of at least approximately 200 V, and wherein a duration of each of the pulses is less than approximately 20 µs.
8. The method according to aspect 7, wherein the sequence of the pulses comprises biphasic pairs of the pulses, wherein each pair comprises a positive pulse and a negative pulse.
9. The method according to aspect 1, wherein applying the pulses comprises applying the pulses in a bipolar mode between one or more of the radial electrodes and the axial electrode.
10. The method according to aspect 1, wherein applying the pulses comprises applying the pulses in a unipolar mode between one or more of the electrodes on the basket assembly, including the axial electrode, and a common electrode that is separate from the probe.
11. The method according to aspect 1, wherein applying the pulses comprises applying the pulses between different pairs of the electrodes sequentially in accordance with a predefined protocol.
12. The method according to aspect 11, wherein in accordance with the predefined protocol, the pulses are applied in a bipolar mode between each of the radial electrodes and the axial electrode.
13. The method according to aspect 12, wherein in accordance with the predefined protocol, the pulses are applied simultaneously between two or more of the radial electrodes and the axial electrode.
14. The method according to aspect 11, wherein in accordance with the predefined protocol, the pulses are applied in a bipolar mode between multiple pairs of the radial electrodes, each pair including a first radial electrode on a first one of the spines and a second radial electrode on a second of the spines, which is not adjacent to the first one or the spines.
15. The method according to aspect 11, wherein in accordance with the predefined protocol, the pulses are applied to the radial electrodes on different ones of the spines in a predefined sequence in which the pulses are not applied in immediate succession to the radial electrodes on mutually adjacent spines.
16. The method according to aspect 11, wherein the radial electrodes comprise at least first and second radial electrodes on each of the spines, and wherein in accordance with the predefined protocol, the pulses are applied to only one of the first and second radial electrodes on each of the spines.

## Claims

1. A medical apparatus, comprising:
a probe, comprising:
an insertion tube (25) configured for insertion into a body cavity of a patient;
a basket assembly (40) having a proximal end that is connected distally to the insertion tube and comprising a plurality of resilient spines (44), which are configured to bow radially outward around a longitudinal axis of the basket assembly and are conjoined at a distal end of the basket assembly; and
a plurality of electrodes, which are configured to contact tissue in the body cavity and comprise radial electrodes disposed on the spines about the longitudinal axis and an axial electrode (50) disposed on the longitudinal axis of the basket assembly;
an electrical signal generator (38) configured to apply to the electrodes, including the axial electrode, pulses having an amplitude sufficient to cause irreversible electroporation (IRE) in the tissue contacted by the electrodes, wherein the electrical signal generator (38) is configured to apply the pulses in accordance with a predefined protocol between different pairs of the electrodes sequentially in a bipolar mode, wherein each pair includes a first radial electrode on a first one of the spines (44) and a second radial electrode on a second of the spines (44), which is not adjacent to the first one of the spines.

2. A medical apparatus, comprising:
a probe, comprising:
an insertion tube (25) configured for insertion into a body cavity of a patient;
a basket assembly (40) having a proximal end that is connected distally to the insertion tube and comprising a plurality of resilient spines (44), which are configured to bow radially outward around a longitudinal axis of the basket assembly and are conjoined at a distal end of the basket assembly; and
a plurality of electrodes, which are configured to contact tissue in the body cavity and comprise radial electrodes disposed on the spines about the longitudinal axis and an axial electrode (50) disposed on the
longitudinal axis of the basket assembly;
an electrical signal generator (38) configured to apply to the electrodes, including the axial electrode, pulses having an amplitude sufficient to cause irreversible electroporation (IRE) in the tissue contacted by the electrodes, wherein the electrical signal generator (38) is configured to apply the pulses between different pairs of the electrodes sequentially in accordance with a predefined protocol, wherein the pulses are applied to the radial electrodes on different ones of the spines in a predefined sequence in which the pulses are not applied in immediate succession to the radial electrodes on mutually adjacent spines.

3. The apparatus according to claim 1 or claim 2, wherein the spines (44) comprise respective proximal and distal tips, wherein the proximal tips of the spines are joined proximate a proximal end of the basket assembly, and the distal tips of the spines are joined to a distal end of the basket assembly, and the spines (44) bow radially outward away from the longitudinal axis when the basket assembly is deployed in the body cavity so that the radial electrodes contact the tissue in the body cavity.

4. The apparatus according to claim 3, wherein the basket assembly (40) comprises a stable collapsed state, and wherein the apparatus comprises a puller (46) attached to the distal end of the basket assembly and slidably disposed within the insertion tube, so that the spines bow radially outward in response to pulling the puller in a proximal direction through the insertion tube.

5. The apparatus according to claim 1 or claim 2, wherein the insertion tube comprises a flexible catheter (22) configured for insertion into a chamber of a heart of the patient, and the electrodes are configured to contact and apply the electrical signals to myocardial tissue within the chamber.

6. The apparatus according to claim 1 or claim 2, wherein the radial electrodes comprise a single respective radial electrode on each of the spines (44).

7. The apparatus according to claim 1 or claim 2, wherein the radial electrodes comprise multiple radial electrodes on each of the spines (44) in respective locations that are longitudinally staggered among the spines.

8. The apparatus according to claim 1 or claim 2, wherein the pulses applied by the electrical signal generator comprise a sequence of the pulses having an amplitude of at least approximately 200 V, and a duration of each of the pulses is less than approximately 20 µs.

9. The apparatus according to claim 8, wherein the sequence of the pulses comprises biphasic pairs of the pulses, wherein each pair comprises a positive pulse and a negative pulse.

10. The apparatus according to claim 1 or claim 2, wherein the electrical signal generator (38) is configured to apply the pulses in a bipolar mode between one or more of the radial electrodes and the axial electrode.

11. The apparatus according to claim 1 or claim 2, wherein the electrical signal generator (38) is configured to apply the pulses in a unipolar mode between one or more of the electrodes on the basket assembly, including the axial electrode, and a common electrode that is separate from the probe.

12. The apparatus according to claim 1 or claim 2, wherein in accordance with the predefined protocol, the pulses are applied in a bipolar mode between each of the radial electrodes and the axial electrode.

13. The apparatus according to claim 12, wherein in accordance with the predefined protocol, the pulses are applied simultaneously between two or more of the radial electrodes and the axial electrode.

## Patentansprüche

1. Medizinische Einrichtung, umfassend:
eine Sonde, umfassend:
einen Einführschlauch (25), der für eine Einführung in eine Körperhöhle eines Patienten konfiguriert ist;
eine Korbanordnung (40), die ein proximales Ende aufweist, das distal mit dem Einführschlauch verbunden ist und umfassend eine Vielzahl von elastischen Dornen (44), die konfiguriert sind, um sich radial nach außen um eine Längsachse der Korbanordnung herum zu biegen und die an einem distalen Ende der Korbanordnung verknüpft sind; und
eine Vielzahl von Elektroden, die konfiguriert sind, um mit Gewebe in der Körperhöhle in Berührung zu kommen, und radiale Elektroden, die auf den Dornen um die Längsachse herum angeordnet sind, und eine axiale Elektrode (50) umfassen, die auf der Längsachse der Korbanordnung angeordnet ist;
einen elektrischen Signalgenerator (38), der konfiguriert ist, um an die Elektroden, einschließlich der axialen Elektrode, Impulse anzulegen, die eine Amplitude aufweisen, die ausreichend ist, um eine irreversible Elektroporation (IRE) in dem Gewebe zu verursachen, das durch die Elektroden berührt wird, wobei der elektrische Signalgenerator (38) konfiguriert ist, um die Impulse gemäß einem vordefinierten Protokoll zwischen verschiedenen Elektrodenpaaren nacheinander in einem bipolaren Modus anzulegen, wobei jedes Paar eine erste radiale Elektrode auf einem ersten der Dorne (44) und eine zweite radiale Elektrode auf einem zweiten der Dorne (44) einschließt, der nicht an den ersten der Dorne angrenzt.

2. Medizinische Einrichtung, umfassend:
eine Sonde, umfassend:
einen Einführschlauch (25), der für eine Einführung in eine Körperhöhle eines Patienten konfiguriert ist;
eine Korbanordnung (40), die ein proximales Ende aufweist, das distal mit dem Einführschlauch verbunden ist und umfassend eine Vielzahl von elastischen Dorne (44), die konfiguriert sind, um sich radial nach außen um eine Längsachse der Korbanordnung herum zu biegen und die an einem distalen Ende der Korbanordnung verknüpft sind; und
eine Vielzahl von Elektroden, die konfiguriert sind, um mit Gewebe in der Körperhöhle in Berührung zu kommen, und radiale Elektroden, die auf den Dornen um die Längsachse herum angeordnet sind, und eine axiale Elektrode (50) umfassen, die auf der Längsachse der Korbanordnung angeordnet ist;
einen elektrischen Signalgenerator (38), der konfiguriert ist, um an die Elektroden, einschließlich der axialen Elektrode, Impulse anzulegen, die eine Amplitude aufweisen, die ausreichend ist, um eine irreversible Elektroporation (IRE) in dem Gewebe zu verursachen, das durch die Elektroden berührt wird, wobei der elektrische Signalgenerator (38) konfiguriert ist, um die Impulse zwischen verschiedenen Elektrodenpaaren nacheinander gemäß einem vordefinierten Protokoll anzulegen, wobei die Impulse an die radialen Elektroden an verschiedenen der Dorne in einer vordefinierten Reihenfolge angelegt werden, in der die Impulse nicht in unmittelbarer Folge an die radialen Elektroden an aneinander angrenzende Dorne angelegt werden.

3. Einrichtung nach Anspruch 1 oder 2, wobei die Dorne (44) jeweilige proximale und distale Spitzen umfassen, wobei die proximalen Spitzen der Dorne nahe einem proximalen Ende der Korbanordnung verknüpft sind und die distalen Spitzen der Dorne mit einem distalen Ende der Korbanordnung verknüpft sind und sich die Dorne (44) radial nach außen weg von der Längsachse biegen, wenn die Korbanordnung in der Körperhöhle derart eingesetzt wird, dass die radialen Elektroden das Gewebe in der Körperhöhle berühren.

4. Einrichtung nach Anspruch 3, wobei die Korbanordnung (40) einen stabilen zusammengefalteten Zustand umfasst, und wobei die Einrichtung einen Abzieher (46) umfasst, der an dem distalen Ende der Korbanordnung befestigt ist und derart gleitbar innerhalb des Einführschlauchs angeordnet ist, dass sich die Dorne als Reaktion auf ein Ziehen des Abziehers in einer proximalen Richtung durch den Einführschlauch radial nach außen biegen.

5. Einrichtung nach Anspruch 1 oder 2, wobei der Einführschlauch einen flexiblen Katheter (22) umfasst, der zum Einführen in eine Kammer eines Herzens des Patienten konfiguriert ist, und die Elektroden konfiguriert sind, um das Herzmuskelgewebe innerhalb der Kammer zu berühren und die elektrischen Signale daran anzulegen.

6. Einrichtung nach Anspruch 1 oder 2, wobei die radialen Elektroden jeweils eine einzelne radiale Elektrode auf jedem der Dorne (44) umfassen.

7. Einrichtung nach Anspruch 1 oder 2, wobei die radialen Elektroden mehrere radiale Elektroden auf jedem der Dorne (44) an jeweiligen Stellen umfassen, die in Längsrichtung zwischen den Dornen versetzt sind.

8. Einrichtung nach Anspruch 1 oder 2, wobei die Impulse, die durch den elektrischen Signalgenerator angelegt werden, eine Folge der Impulse umfassen, die eine Amplitude von mindestens etwa 200 V aufweisen und eine Dauer jedes der Impulse weniger als etwa 20 µs beträgt.

9. Einrichtung nach Anspruch 8, wobei die Folge der Impulse zweiphasige Paare der Impulse umfasst, wobei jedes Paar einen positiven Impuls und einen negativen Impuls umfasst.

10. Einrichtung nach Anspruch 1 oder 2, wobei der elektrische Signalgenerator (38) konfiguriert ist, um die Impulse in einem bipolaren Modus zwischen einer oder mehreren der radialen Elektroden und der axialen Elektrode anzulegen.

11. Einrichtung nach Anspruch 1 oder 2, wobei der elektrische Signalgenerator (38) konfiguriert ist, um die Impulse in einem unipolaren Modus zwischen einer oder mehreren der Elektroden auf der Korbanordnung, einschließlich der axialen Elektrode, und einer gemeinsamen Elektrode anzulegen, die von der Sonde getrennt ist.

12. Einrichtung nach Anspruch 1 oder 2, wobei die Impulse gemäß dem vordefinierten Protokoll in einem bipolaren Modus zwischen jeder der radialen Elektroden und der axialen Elektrode angelegt werden.

13. Einrichtung nach Anspruch 12, wobei die Impulse gemäß dem vordefinierten Protokoll gleichzeitig zwischen zwei oder mehreren der radialen Elektroden und der axialen Elektrode angelegt werden.

## Revendications

1. Appareil médical comprenant :
une sonde, comprenant :
un tube d'insertion (25) conçu pour être inséré dans une cavité corporelle d'un patient ;
un ensemble panier (40) présentant une extrémité proximale qui est reliée de manière distale au tube d'insertion et comprenant une pluralité d'épines (44) élastiques conçues pour s'incliner radialement vers l'extérieur autour d'un axe longitudinal de l'ensemble panier et qui sont raccordées à une extrémité distale de l'ensemble panier ; et
une pluralité d'électrodes, configurées pour entrer en contact avec le tissu dans la cavité corporelle et comprenant des électrodes radiales disposées sur les épines autour de l'axe longitudinal et une électrode axiale (50) disposée sur l'axe longitudinal de l'ensemble panier ;
un générateur de signaux électriques (38) configuré pour appliquer aux électrodes, y compris à l'électrode axiale, des impulsions ayant une amplitude suffisante pour provoquer une électroporation irréversible (IRE) dans le tissu mis en contact par les électrodes, dans lequel le générateur de signaux électriques (38) est configuré pour appliquer les impulsions conformément à un protocole prédéfini entre différentes paires des électrodes de manière séquentielle dans un mode bipolaire, dans lequel chaque paire comporte une première électrode radiale sur une première épine des épines (44) et une seconde électrode radiale sur une seconde des épines (44), qui n'est pas adjacente à la première des épines.

2. Appareil médical comprenant :
une sonde, comprenant :
un tube d'insertion (25) conçu pour être inséré dans une cavité corporelle d'un patient ;
un ensemble panier (40) présentant une extrémité proximale qui est reliée de manière distale au tube d'insertion et comprenant une pluralité d'épines (44) élastiques conçues pour s'incliner radialement vers l'extérieur autour d'un axe longitudinal de l'ensemble panier et qui sont raccordées à une extrémité distale de l'ensemble panier ; et
une pluralité d'électrodes, configurées pour entrer en contact avec le tissu dans la cavité corporelle et comprenant des électrodes radiales disposées sur les épines autour de l'axe longitudinal et une électrode axiale (50) disposée sur l'axe longitudinal de l'ensemble panier ;
un générateur de signaux électriques (38) configuré pour appliquer aux électrodes, y compris à l'électrode axiale, des impulsions ayant une amplitude suffisante pour provoquer une électroporation irréversible (IRE) dans le tissu mis en contact par les électrodes, dans lequel le générateur de signaux électriques (38) est configuré pour appliquer les impulsions entre différentes paires des électrodes de manière séquentielle conformément à un protocole prédéfini, dans lequel les impulsions sont appliquées aux électrodes radiales sur différentes épines des épines dans une séquence prédéfinie dans laquelle les impulsions ne sont pas appliquées en succession immédiate aux électrodes radiales sur des épines mutuellement adjacentes.

3. Appareil selon la revendication 1 ou la revendication 2, dans lequel les épines (44) comprennent des pointes proximales et distales respectives, dans lequel les pointes proximales des épines sont raccordées à proximité d'une extrémité proximale de l'ensemble panier, et les extrémités pointes distales des épines sont raccordées à une extrémité distale de l'ensemble panier, et les épines (44) s'inclinent radialement vers l'extérieur à partir de l'axe longitudinal lorsque l'ensemble panier est déployé dans la cavité corporelle de sorte que les électrodes radiales entrent en contact avec le tissu dans la cavité corporelle.

4. Appareil selon la revendication 3, dans lequel l'ensemble panier (40) comprend un état affaissé stable, et dans lequel l'appareil comprend un extracteur (46) fixé à l'extrémité distale de l'ensemble panier et disposé de manière coulissante à l'intérieur du tube d'insertion, de sorte que les épines s'inclinent radialement vers l'extérieur en réponse à la traction de l'extracteur dans une direction proximale à travers le tube d'insertion.

5. Appareil selon la revendication 1 ou la revendication 2, dans lequel le tube d'insertion comprend un cathéter (22) souple conçu pour être inséré dans une chambre du cœur du patient, et les électrodes sont configurées pour entrer en contact avec le tissu myocardique à l'intérieur de la chambre et y appliquer les signaux électriques.

6. Appareil selon la revendication 1 ou la revendication 2, dans lequel les électrodes radiales comprennent une seule électrode radiale respective sur chacune des épines (44).

7. Appareil selon la revendication 1 ou la revendication 2, dans lequel les électrodes radiales comprennent de multiples électrodes radiales sur chacune des épines (44) à des emplacements respectifs qui sont longitudinalement décalés parmi les épines.

8. Appareil selon la revendication 1 ou la revendication 2, dans lequel les impulsions appliquées par le générateur de signaux électriques comprennent une séquence des impulsions présentant une amplitude d'au moins approximativement 200 V et une durée de chacune des impulsions est inférieure à approximativement 20 µs.

9. Appareil selon la revendication 8, dans lequel la séquence des impulsions comprend des paires d'impulsions biphasiques, dans lequel chaque paire comprend une impulsion positive et une impulsion négative.

10. Appareil selon la revendication 1 ou la revendication 2, dans lequel le générateur de signaux électriques (38) est configuré pour appliquer les impulsions dans un mode bipolaire entre une ou plusieurs des électrodes radiales et l'électrode axiale.

11. Appareil selon la revendication 1 ou la revendication 2, dans lequel le générateur de signaux électriques (38) est configuré pour appliquer les impulsions dans un mode unipolaire entre une ou plusieurs des électrodes sur l'ensemble panier, y compris l'électrode axiale, et une électrode commune qui est séparée de la sonde.

12. Appareil selon la revendication 1 ou la revendication 2, dans lequel conformément au protocole prédéfini, les impulsions sont appliquées dans un mode bipolaire entre chacune des électrodes radiales et l'électrode axiale.

13. Appareil selon la revendication 12, dans lequel, conformément au protocole prédéfini, les impulsions sont appliquées simultanément entre deux ou plusieurs des électrodes radiales et l'électrode axiale.
